# EUROPÄISCHE PATENTSCHRIFT

(11) **EP 2 122 365 B1**
(45) Veröffentlichungstag und Bekanntmachung des Hinweises auf die Patenterteilung: **19.12.2012**
(21) Anmeldenummer: 07856155.2
(22) Anmeldetag: 22.12.2007
(51) Int. Cl.: G01N 33/68

(54) **DIAGNOSE UND RISIKOSTRATIFIZIERUNG DES AKUTEN KORONARSYNDROMS MITTELS CT-PROET-1 IN KOMBINATION MIT NT-PROBNP**
DIAGNOSIS AND RISK CLASSIFICATION OF ACUTE CORONARY SYNDROME BY MEANS OF CT-PROET-1 IN COMBINATION WITH NT-PROBNP
DIAGNOSTIC ET CLASSEMENT DES RISQUES DU SYNDROME CORONARIEN AIGU A L'AIDE DU CT-PROET-1 EN COMBINAISON AVEC LA NT-PROBNP

(30) Priorität: 22.12.2006 DE 102006060835
(43) Veröffentlichungstag der Anmeldung: 25.11.2009
(73) Patentinhaber: B.R.A.H.M.S GmbH, 16761 Hennigsdorf (DE)
(72) Erfinder: BERGMANN, Andreas, 12351 Berlin (DE); MORGENTHALER, Nils, 13503 Berlin (DE); PAPASSOTIRIOU, Jana, 14163 Berlin (DE); STRUCK, Joachim, 13465 Berlin (DE); NG, Leong L., Leicester LE2 3NB (GB)
(74) Vertreter: Simandi, Claus
(86) Internationale Anmeldenummer: PCT/DE2007/002313
(87) Internationale Veröffentlichungsnummer: WO 2008/077396

(56) Entgegenhaltungen:
- EP-A- 1 363 128
- EP-A- 1 619 505
- WO-A-2005/078456
- KHAN SOHAIL Q ET AL: "C-terminal pro-endothelin-1 offers additional prognostic information in patients after acute myocardial infarction: Leicester Acute Myocardial Infarction Peptide (LAMP) Study." AMERICAN HEART JOURNAL OCT 2007, Bd. 154, Nr. 4, Oktober 2007 (2007-10), Seiten 736-742, XP002481913 ISSN: 1097-6744

### SEQUENCE LISTING

<110> BRAHMS Aktiengesellschaft
<120> Diagnose und Risikostratifizierung des akuten Koronarsyndroms mittels CT-proET-1 in Kombination mit NT-proBNP
<130> BRAHMS33WO
<140> PCT/DE2007/
   <141> 2007-12-22
<150> DE 10 2006 060 835.6
   <151> 2006-12-22
<160> 4
<170> PatentIn version 3.3
<210> 1
   <211> 212
   <212> PRT
   <213> Human
<400> 1
   Met Asp Tyr Leu Leu Met Ile Phe Ser Leu Leu Phe Val Ala Cys Gln
   1               5                   10                  15
   Gly Ala Pro Glu Thr Ala Val Leu Gly Ala Glu Leu Ser Ala Val Gly
               20                  25                  30
   Glu Asn Gly Gly Glu Lys Pro Thr Pro Ser Pro Pro Trp Arg Leu Arg
           35                  40                  45
   Arg Ser Lys Arg Cys Ser Cys Ser Ser Leu Met Asp Lys Glu Cys Val
       50                  55                  60
   Tyr Phe Cys His Leu Asp Ile Ile Trp Val Asn Thr Pro Glu His Val
   65                  70                  75                  80
   Val Pro Tyr Gly Leu Gly Ser Pro Arg Ser Lys Arg Ala Leu Glu Asn
                   85                  90                  95
   Leu Leu Pro Thr Lys Ala Thr Asp Arg Glu Asn Arg Cys Gln Cys Ala
               100                 105                 110
   Ser Gln Lys Asp Lys Lys Cys Trp Asn Phe Cys Gln Ala Gly Lys Glu
           115                 120                 125
   Leu Arg Ala Glu Asp Ile Met Glu Lys Asp Trp Asn Asn His Lys Lys
       130                 135                 140
   Gly Lys Asp Cys Ser Lys Leu Gly Lys Lys Cys Ile Tyr Gln Gln Leu
   145                 150                 155                 160
   Val Arg Gly Arg Lys Ile Arg Arg Ser Ser Glu Glu His Leu Arg Gln
                   165                 170                 175
   Thr Arg Ser Glu Thr Met Arg Asn Ser Val Lys Ser Ser Phe His Asp
   180 185 190
   Pro Lys Leu Lys Gly Lys Pro Ser Arg Glu Arg Tyr Val Thr His Asn
   195 200 205
   Arg Ala His Trp
   210
<210> 2
   <211> 120
   <212> PRT
   <213> Human
<400> 2
   Ala Leu Glu Asn Leu Leu Pro Thr Lys Ala Thr Asp Arg Glu Asn Arg
   1 5 10 15
   Cys Gln Cys Ala Ser Gln Lys Asp Lys Lys Cys Trp Asn Phe Cys Gln
   20 25 30
   Ala Gly Lys Glu Leu Arg Ala Glu Asp Ile Met Glu Lys Asp Trp Asn
   35 40 45
   Asn His Lys Lys Gly Lys Asp Cys Ser Lys Leu Gly Lys Lys Cys Ile
   50 55 60
   Tyr Gln Gln Leu Val Arg Gly Arg Lys Ile Arg Arg Ser Ser Glu Glu
   65 70 75 80
   His Leu Arg Gln Thr Arg Ser Glu Thr Met Arg Asn Ser Val Lys Ser
   85 90 95
   Ser Phe His Asp Pro Lys Leu Lys Gly Lys Pro Ser Arg Glu Arg Tyr
   100 105 110
   Val Thr His Asn Arg Ala His Trp
   115 120
<210> 3
   <211> 45
   <212> PORT
   <213> Human
<400> 3
   Arg Ser Ser Glu Glu His Leu Arg Gln Thr Arg Ser Glu Thr Met Arg
   1 5 10 15
   Asn Ser Val Lys Ser Ser Phe His Asp Pro Lys Leu Lys Gly Lys Pro
   20 25 30
   Ser Arg Glu Arg Tyr Val Thr His Asn Arg Ala His Trp
   35 40 45
<210> 4
   <211> 76
   <212> PRT
   <213> Human
<400> 4
   His Pro Leu Gly Ser Pro Gly Ser Ala Ser Asp Leu Glu Thr Ser Gly
   1 5 10 15
   Leu Gln Glu Gln Arg Asn His Leu Gln Gly Lys Leu Ser Glu Leu Gln
   20 25 30
   Val Glu Gln Thr Ser Leu Glu Pro Leu Gln Glu Ser Pro Arg Pro Thr
   35 40 45
   Gly Val Trp Lys Ser Arg Glu Val Ala Thr Glu Gly Ile Arg Gly His
   50 55 60
   Arg Lys Met Val Leu Tyr Thr Leu Arg Ala Pro Arg
   65 70 75

## Beschreibung

Die Erfindung betrifft ein Verfahren zur Diagnose und / oder Risikostratifizierung des akuten Koronarsyndroms (ACS), insbesondere des akuten Myokardinfarkts (AMI) und der Angina pectoris (AP) und / oder eines post-Myokardinfarkts (post-AMI), wobei eine Bestimmung des C-terminalen proEndothelins (CT-proET-1) in Kombination mit NT-proBNP erfolgt.

Die Risikostratifizierung hat eine zunehmende Bedeutung im Bereich der Herzerkrankungen, sei es bei symptomatischen oder asymptomatischen Patienten. Insbesondere auf dem Gebiet des akuten Koronarsyndroms (ACS) sowie des akuten Myokardinfarkts (AMI) und der Angina pectoris (AP) sowie des post-Myokardinfarkts (post-AMI) besteht ein hohes Bedürfnis an einer geeigneten Risikostratifizierung.

Zwecks einer geeigneten Therapie, bedarf es einer frühen Diagnose und Differenzierung des akuten Koronarsyndroms bereits in der Notaufnahme in Verbindung mit der Notwendigkeit klinische Entscheidungen zu treffen. Aufgrund unspezifischer Symptome (Brustschmerzen) bei akutem Koronarsyndrom ist sowohl die Differenzierung und Abgrenzung von anderen Erkrankungen als auch die Erkennung des akuten Koronarsyndroms wesentlich. Dies ist ebenfalls erforderlich im Fall eines post-Myokardinfarktes.

Im Stand der Technik sind biochemische Marker - insbesondere die Klassiker wie kardiale Troponine, Myoglobin und CK-MB-Masse- zur Prognose eines Myokardinfarkts angestrengt worden (Katus, H. A.; Remppis, A.; Scheffold, T.; Diederich, K. W. und Kuebler, W. (1991): Intracellular compartmentation of cardiac troponin T and its release kinetics in patients with reperfused and nonreperfused myocardial infarction, Am J Cardiol 67 (16): 1360-1367). Als ein weiterer effektiver biochemischer Marker hat sich in der Myokarddiagnostik das B-Typ natriuretischem Peptid (BNP) samt Pro-BNP, NT-ProBNP (EP1363128B1, EP1666881A2) erwiesen.

Die C-terminalen ProEndothelin Fragmente (CT-proET-1) mit den Aminosäuresequenzen 93-212 oder 168-212 des preproEndothelin (SEQ ID No. 1) ist zur Diagnose von Herz-Kreislauferkrankungen zur indirekten Bestimmung des Endothelin-1 oder des big - Endothelin-1 Gehaltes in EP 1564558 B1 beschrieben.

Ferner ist aus EP 1564558 B1 bekannt, dass zur Kardialdiagnostik Kardialparameter wie ANP, BNP und proBNP in einer Multiparameterdiagnostik hinzugezogen werden können.

Nachteilig an den bekannten Diagnoseverfahren unter Verwendung der bisher bekannten Marker ist jedoch, dass eine frühzeitige und vollständige Erfassung von Risikopatienten nicht ausreichend gelingt und daher eine Risikostratifizierung nicht ausreichend erfolgt. Eine der Erfindung zugrunde liegende Aufgabe besteht daher darin, ein Verfahren zur Risikostratifizierung des akuten Koronarsyndroms sowie des post-Myokardinfarktes zu entwickeln, das eine verbesserte Erfassung von Risikopatienten ermöglicht.

Ferner ist nachteilig, dass im Stand der Technik zumeist keine hinreichende Sensitivität und/oder Spezifität der Marker erreicht wird.

Eine weitere Aufgabe besteht daher darin, ein Verfahren zur Risikostratifizierung des akuten Koronarsyndroms sowie des post-Myokardinfarktes bereitzustellen, wobei mindestens ein Marker oder eine Kombination von Markern eine hinreichende Sensitivität und Spezifität in einer in-vitro Diagnose aufweist.

Daher ist es Aufgabe der vorliegenden Erfindung ein Verfahren zur Diagnose und / oder Risikostratifizierung des akuten Koronarsyndroms sowie eines post-Myokardinfarktes bereitzustellen.

Die Aufgabe wird durch ein Verfahren zur Diagnose und Risikostratifizierung des akuten Koronarsyndroms sowie eines post-Myokardinfarktes gelöst, wobei eine Bestimmung des CT-proET-1 mit den freien Fragmenten (SEQ ID No. 2 und / oder SEQ ID No. 3) (kurz: "CT-proET-1"), in Kombination mit NT-proBNP (SEQ ID No. 4) erfolgt (nachstehend erfindungsgemäßes Verfahren).

Überraschender Weise weist CT-proET-1 in Kombination mit NT-proBNP eine hohe Sensitivität und Spezifität für die Diagnose des akuten Koronarsyndroms sowie des post-Myokardinfarkts auf (Siehe Beispiele und Figuren).

Der Begriff "akutes Koronarsyndrom" umfasst verschiedene Phasen der koronaren Herzerkrankung, die unmittelbar lebensbedrohlich sind. Dies betrifft insbesondere die Notfallmedizin, und zwar einen akuten Myokardinfarkt und /oder Angina pectoris sowie einen plötzlichen Herztod. Neben dem akuten Myokardinfarkt, der nach WHO-Kriterien (WHO (1979): Nomenclature and criteria for diagnosis of ischemic heart disease. Report of the Joint International Society and Federation of Cardiology/World Health-Organization task force on standardization of clinical nomenclature, Circulation 59 (3): 607-609) definiert ist als akutes, länger als 20 Minuten andauerndes Brustschmerzereignis, verbunden mit ST-Streckenhebungen und/oder einer Erhöhung myokardialer Enzyme, wurde der Begriff der instabilen Angina pectoris (AP) geprägt, die erfindungsgemäß unter "akutes Koronarsyndrom" zu lesen sind (Hamm CW: Leitlinien: Akutes Koronarsyndrom (ACS) - Teil 1: ACS ohne persistierende ST-Hebung. Z Kardiol (2004) 93:72-90; siehe auch: Pschyrembel, De Gruyter, Berlin 2004). Im Rahmen dieser Erfindung wird unter "nachteiliges (negatives) Ereignis bei post-Myokardinfarktpatienten" insbesondere ein weiterer (Folge-) Myokardinfarkt, Herzversagen oder Todeseintritt verstanden oder sonstige Verschlechterung der Prognose des Patienten verstanden.

Im Rahmen dieser Erfindung wird unter "Myokardinfarkt" (Herzinfarkt, AMI (acute myocardial infarction)) eine akute und lebensbedrohliche Erkrankung des Herzens verstanden, wobei ein Absterben oder Gewebsuntergang (Infarkt) von Teilen des Herzmuskels (Myokard) erfolgt, infolge einer Durchblutungsstörung (Ischämie), die in der Regel länger als 20 Minuten besteht. Leitsymptom des Herzinfarktes ist ein plötzlich auftretender, mehr als 20 Minuten anhaltender und meist starker Schmerz im Brustbereich ("chest pain"), der in die Schultern, Arme, Unterkiefer und Oberbauch ausstrahlen kann und von Schweißausbrüchen, Übelkeit und evtl. Erbrechen begleitet sein kann. Infolge eines Myokardinfarktes ist ein Herzversagen möglich.

Der Begriff "post-Myokardinfarkt" bedeutet, dass ein Patient bereits einen Myokardinfarkt in der Vergangenheit, d.h. z.B. mehr als 1 Stunde, insbesondere 20 Stunden, insbesondere 1 bis 5 Tage oder 3 bis 5 Tage erlitten hat und nunmehr in der Post ("Danach") - Phase lebt und keine unmittelbare Todesfolge erlitten hat, jedoch mittelbar und unmittelbar mit einem weiteren nachteiligen Ereignis gerechnet werden kann.

Alle genannten Indikationen werden zudem z.B. im Pschyrembel, De Gruyter, Berlin 2004 beschrieben.

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung des akuten Koronarsyndroms, insbesondere Myokardinfarkt, Angina pectoris und / oder post-Myokardinfarkt mit dem Ziel eines möglichst günstigen Verlaufs zu ermöglichen. Eine erfindungsgemäße Risikostratifizierung erlaubt in Folge ein effektives Behandlungsverfahren, die beim akuten Koronarsyndrom mit den perkutanen Koronarinterventionen und den neueren Arzneimitteln gegeben sind.

Daher betrifft die Erfindung ebenfalls die Identifizierung von Patienten mit erhöhtem Risiko oder/und einer ungünstigen Prognose eines akuten Koronarsyndroms, insbesondere Myokardinfarkts, Angina pectoris und / oder post-Myokardinfarkt und zwar bei symptomatischen und / oder asymptomatischen Patienten, insbesondere Notfallpatienten.

Besonders vorteilhaft kann insbesondere in Fällen der Notfall- und /oder Intensivmedizin mittels des erfindungsgemäßen Verfahrens eine sichere Stratifizierung erfolgen. Das erfindungsgemäße Verfahren ermöglicht daher klinische Entscheidungen, die zu einem schnellen Therapieerfolg und zur Vermeidung von Todesfällen führen. Solche klinischen Entscheidungen umfassen ebenfalls weiterführende Behandlungen mittels Arzneimitteln zur Behandlung oder Therapie des akuten Koronarsyndroms, insbesondere Myokardinfarkt (AMI) und der Angina pectoris (AP) und / oder post-Myokardinfarkt.

Daher betrifft die Erfindung ebenfalls ein Verfahren zur Diagnose und / oder Risikostratifizierung von Patienten mit einem akuten Koronarsyndrom und / oder post-Myokardinfarkt zur Durchführung von klinischen Entscheidungen, wie weiterführende Behandlung und Therapie mittels Arzneimitteln, vorzugsweise in der zeitlich kritischen Intensivmedizin oder Notfallmedizin, einschließlich der Entscheidung zur Hospitalisierung des Patienten.

In einer weiteren bevorzugten Ausführungsform betrifft das erfindungsgemäße Verfahren daher die Therapiesteuerung des akuten Koronarsyndroms, insbesondere Myokardinfarkt (AMI) und der Angina pectoris (AP) sowie post-Myokardinfarkt.

In einer weiteren bevorzugten Ausführungsform des erfindungsgemäßen Verfahrens erfolgt die zur Diagnose und /oder Risikostratifizierung zur Prognose, zur differentialdiagnostischen Früherkennung und Erkennung, zur Beurteilung des Schweregrades und zur therapiebegleitenden Verlaufsbeurteilung.

In einer weiteren bevorzugten Ausführungsform betrifft die Erfindung ein Verfahren zur in-vitro Diagnostik zur Früh- oder Differentialdiagnose oder Prognose des akuten Koronarsyndrom bzw. Myokardinfarkt oder Angina Pectoris und / oder post-Myokardinfarkt, wobei eine Bestimmung des Marker CT-proET-1 in Kombination mit NT-proBNP an einem zu untersuchenden Patienten durchgeführt wird.

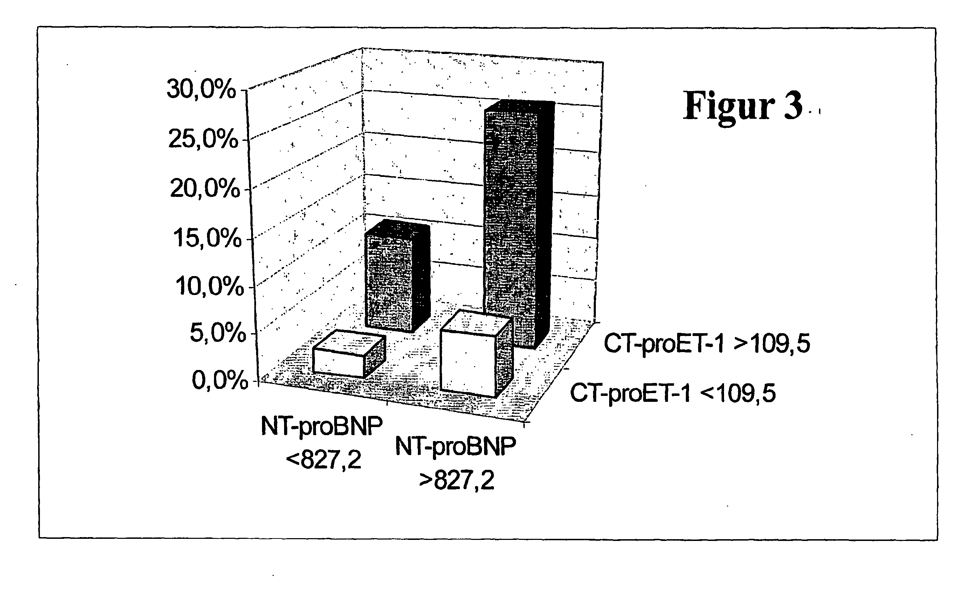

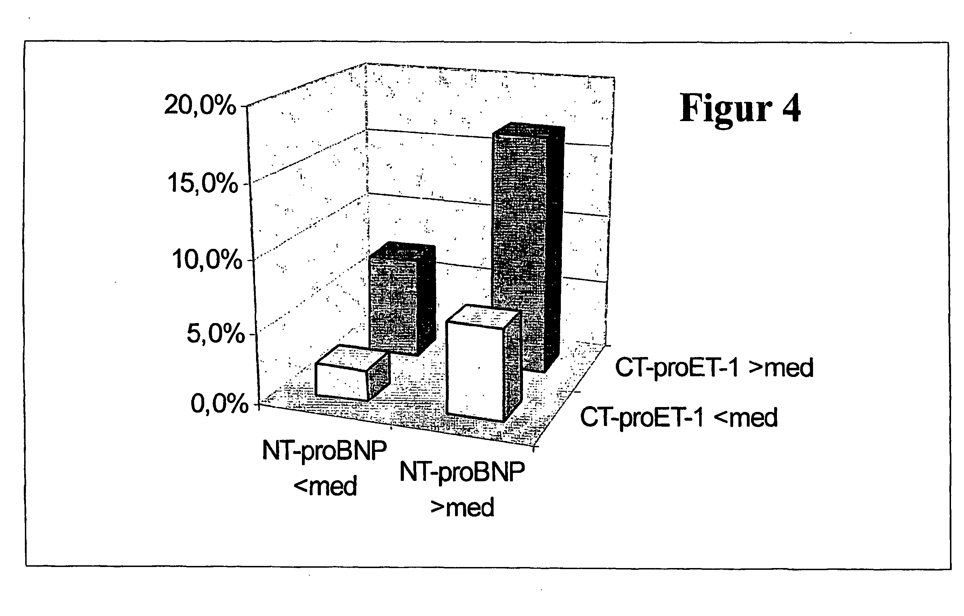

Ferner betrifft die Erfindung ein Verfahren zur Diagnose und Risikostratifizierung des akuten Koronarsyndroms oder ein Verfahren zur Früh- oder Differentialdiagnose oder Prognose von akutem Koronarsyndrom, insbesondere Myokardinfarkt oder Angina Pectoris und / oder post-Myokardinfarkt nach einem der obigen Ausführungsformen, wobei nach Eintreten der Symptome für die Kombination von CT-proET-1 mit NT-proBNP bei cut-off-Werten (Schwellenwerten) von 80-150 pmol/L, insbesondere 90 - 130 pmol/L insbesondere 109,5 pmol/L für CT-proET-1 und 750 - 1100 pmol/L, insbesondere 800 - 950 pmol/L, insbesondere 827,2 pmol/L für NT-proBNP bei maximaler Sensitivität und Spezifität (siehe Figuren 3 und 4) für die Diagnose und / oder Risikostratifizierung in den genannten Ausführungsformen betragen.

Auf dieser Basis sind diese erfindungsgemäßen Verfahren vorteilhaft sensitiv und spezifisch.

In einer Ausführungsform des erfindungsgemäßen Verfahrens wird dem zu untersuchenden Patienten Körperflüssigkeit, vorzugsweise Blut entnommen, wahlweise Vollblut oder Serum oder erhältliches Plasma und die Diagnose erfolgt *in vitro*/*ex vivo,* d.h. außerhalb des menschlichen oder tierischen Körpers. Aufgrund der Bestimmung des Markers CT-proET-1 in Kombination mit NT-proBNP wird eine hohe Sensitivität und Spezifität für das akute Koronarsyndrom, Myokardinfarkt und Angina Pectoris und / oder post-Myokardinfarkt erzielt und anhand der vorhandenen Menge in mindestens einer Patientenprobe kann die Diagnose oder Risikostratifizierung erfolgen.

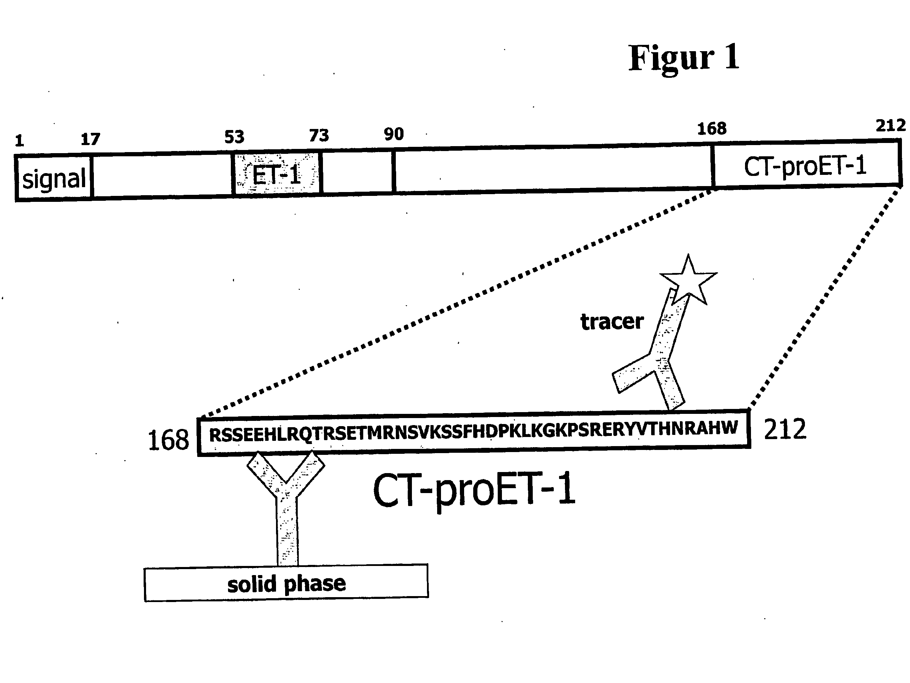

Im Rahmen dieser Erfindung wird unter "CT-proET-1" ein humanes Protein oder Polypeptid verstanden, dass aus dem Preproendothelin erhalten werden kann und im Rahmen des Preproendothelins (SEQ ID No.1) die freien Fragmente mit den Aminosäuren 93-212 (129 AS, SEQ ID No. 2: ALENLLPT KATDRENRCQ CASQKDKKCW NFCQAGKELR AEDIMEKDWN NHKKGKDCSK LGKKCIYQQL VRGRKIRRSS EEHLRQTRSE TMRNSVKSSF HDPKLKGKPS RERYVTHNRA HW) oder mit den Aminosäuren 168-212 (44AS, SEQ ID No. 3: RSS EEHLRQTRSE TMRNSVKSSF HDPKLKGKPS RERYVTHNRA HW, siehe auch Figur 1) umfassen. Ferner können diese erfindungsgemäße Polypeptide posttranslationale Modifikationen aufweisen, wie Glykolisierung, Lip(o)idisierung oder Derivatisierungen.

Im Rahmen dieser Erfindung wird unter "NT-proBNP" das N-termimale Prohormon des B-Typ natriuretischem Peptids ein humanes Protein oder Polypeptid verstanden mit der Aminosäureseguenz gemäß SEQ ID No. 4 (76 AS: HPLG SPGSASDLET SGLQEQRNHL QGKLSELQVE QTSLEPLQES PRPTGVWKSR EVATEGIRGH RKMVLYTLRA PR).

In einer weiteren Ausführungsform kann die Bestimmung von CT-proET-1 in Kombination mit NT-proBNP zusätzlich mit weiteren Markern erfolgen und zwar vorzugsweise solche, die bereits auf ein akutes Koronarsyndrom, insbesondere Myokardinfarkt oder Angina Pectoris sowie post-Myokardinfarkt hinweisen.

Daher betrifft die Erfindung eine solche Ausführungsform des erfindungsgemäßen Verfahrens, wobei die Bestimmung zusätzlich zu der erfindungsgemäßen kombination mit mindestens einem weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker, insbesondere ein Nekrose von Herzmuskelgewebe anzeigender Marker und den Blutdruck beeinflussender Marker aus mindestens einem Marker aus der Gruppe Kreatinkinase, Myoglobin, Myeloperoxidase, weiteres natriuretisches Protein, insbesondere ANP (bzw. ANF), proANP, NT-proANP, BNP, proBNP oder jeweils eine Teilsequenz davon, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Leptin, pro-Neuropeptid-Y, pro-Somatostatin, pro- Neuropeptid-YY, pro-Opiomelanocortin oder pro-Adrenomedullin (proADM) oder jeweils eine Teilsequenz davon.

Der ischämische Marker kann aus mindestens einem Marker aus der Gruppe Troponin I und T, CK-MB ausgewählt sein. Darüber hinaus kann der neurohormonale Marker mindestens ein weiteres natriuretisches Protein sein, insbesondere ANP (bzw. ANF), proANP, NT-proANP, BNP, proBNP oder jeweils eine Teilsequenz davon.

In einer weiteren Ausführungsform der Erfindung kann das erfindungsgemäße Verfahren im Rahmen einer in-vitro Diagnose mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen in einer diagnostischen Vorrichtung anhand eines Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test oder Point-of Care), sei es in Einzel- oder Multiparameterbestimmung. In einer besonders bevorzugten Ausführungsform handelt es sich um einen Selbsttest oder um eine Vorrichtung, die in der Notfalldiagnostik geeignet ist.

Ferner betrifft die Erfindung die Verwendung von CT-proET-1 in Kombination mit NT-proBNP zur Risikostratifizierung des akuten Koronarsyndroms, Myokardinfarkt oder Angina Pectoris und / oder post-Myokardinfarkt und/oder zur in-vitro Diagnostik zur Früh- oder Differentialdiagnose oder Prognose des akuten Koronarsyndroms, Myokardinfarkt oder Angina Pectoris und / oder post-Myokardinfarkt.

Die Erfindung betrifft zudem ein Kit zur Diagnose und / oder Risikostratifizierung des akuten Koronarsyndroms, Myokardinfarkt und / oder Angina Pectoris und / oder post-Myokardinfarkt, enthaltend Nachweisreagenzien zur Bestimmung von CT-proET-1 in Kombination mit NT-proBNP ggfs. oben genannte weitere Marker. Solche Nachweisreagenzien umfassen z.B. Antikörper etc. Nachfolgende Beispiele und Figuren dienen zur näheren Erläuterung der Erfindung, jedoch ohne die Erfindung auf diese Beispiele und Figuren zu beschränken.

### Beispiele und Figuren:

### Beispiel 1

983 Patienten, die sich mit dem Herzinfarkt-bedingten Leitsymptom des Brustschmerzes in der Notaufnahme eines Krankenhauses vorgestellt haben, wurde eine Blutprobe entnommen.

Die Patienten wurden im Median über 342 Tage beobachtet, wobei 10,3% der Patienten starben und 5,0% wegen Herzinsuffizienz rehospitalisiert wurden.

Die Plasma Konzentrationen von CT-proET-1 bei Patienten mit Herzinfarkt waren gegenüber dem Normalbereich (Mittelwert: 44,3 pmol/L, Bereich 10.5-77, 4 pmol/L) mit im Median 71 pmol/L Bereich 4,6-671 pmol/L) erhöht. Im Vergleich zu Patienten ohne spätere Ereignisse war CT-proET-1 höher bei Patienten, die später starben oder mit Herzinsuffizienz rehospitalisiert wurden (Median [Bereich] pmol/L, 123,5; [14-671] vs. 74; [4,6-530; p<0.0001). Auch NT-proBNP war bei Patienten mit Ereignissen gegenüber Patienten ohne spätere Ereignisse erhöht (p<0.0001). Weitere Einzelheiten sind in den Tabellen 1 und 2 dargelegt.

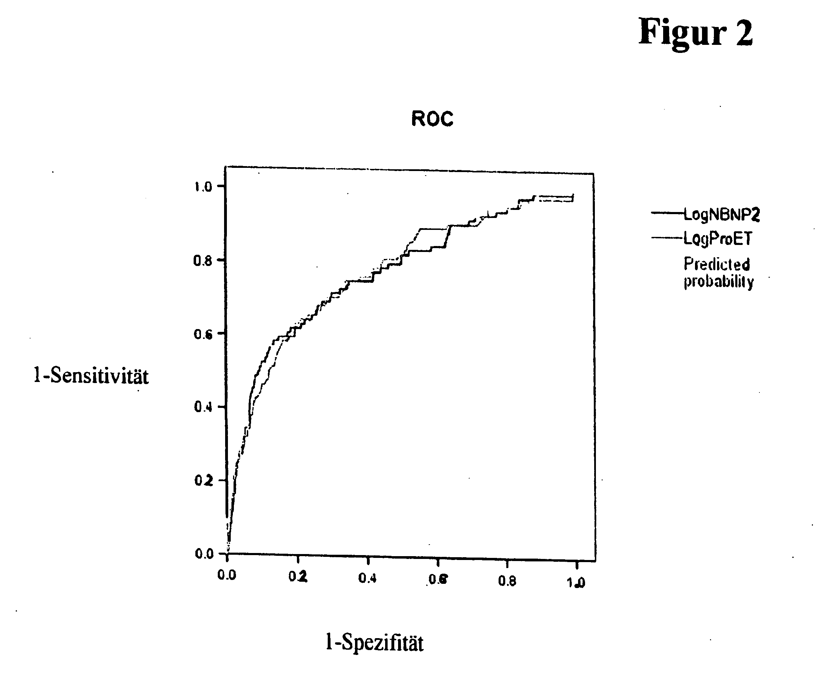

Das Cox proportional hazards model für die Vorhersage von Tod/Herzinsuffizienz identifizierte CT-proET-1 und NT-proBNP als unabhängige Prädiktoren (log CT-proET-1 (HR 6,82), log NT-BNP (HR 2,66)). Die receiver-operating curve für CT-proET-1 erbrachte eine area under the curve (AUC) von 0,77(95% CI: 0,71-0,83, p<0.001); für NT-proBNP war die AUC ebenfalls 0,77 (95% CI: 0,71-0,83, p<0.001); die Kombination beider Marker in dem logistischen Modell erbrachte eine AUC von 0,82 (95% CI: 0,77-0,87, p<0,001), also eine größere AUC als diejenigen der beiden einzelnen Marker (Figur 2).

Der Zusatznutzen, der aus der Kombination beider Marker CT-proET-1 und NT-proBNP gegenüber beiden einzelnen Markern resultiert, ist ebenfalls in den Figuren 3 und 4 veranschaulicht, wobei die Ereignisrate nach 60 Tagen zugrunde gelegt wurde. Patienten wurden nach cut-off-Werten stratifiziert und die Ereignisrate in den so erhaltenen Subgruppen dargestellt. In Figur 3 wurden als cut-off-Werte diejenigen Konzentrationen aus der ROC-Analyse ermittelt, bei denen das Produkt aus Sensitivität und Spezifität für die Ereignisprognose maximal war. In Figur 4 dienen die Mediane der gesamten Patientenpopulation als Cut-off-Wert.

**Tabelle 1: Charakteristik der Patienten, separiert in CT-proET-1 Quartilen. Werte sind als Mittelwerte (SD) oder Zahlen(%) angegeben.**

| | 1. Quartile | 2. Quartile | 3. Quartile | 4. Quartile | p Wert |
|---|---|---|---|---|---|
| Alter (in Jahren) | 58.9 ± 11.7 | 63.5 ± 11.7 | 65.9 ± 11.3 | 71.7 ± 10.7 | <0.0001 |
| Männer | 202 (84.2) | 184 (75.7) | 168 (70.0) | 158 (64.8) | <0.0001 |
| Medizinische | | | | | |
| Vorgeschichte | | | | | |
| akuter Myokardinfarkt | 26 (10.8) | 40 (16.5) | 38 (15.8) | 60 (24.6) | 0.001 |
| Angina Pectoris | 56 (23.3) | 62 (25.5) | 52 (21.7) | 79 (32.4) | 0.038 |
| Hypertension | 82 (24.2) | 106 (43.6) | 101 (42.1) | 131(53.7) | <0.0001 |
| Diabetes mellitus | 34 (14.2) | 47 (19.3) | 51 (21.3) | 79 (32.4) | <0.0001 |
| Hohes Cholesterol | 47 (19.6) | 52 (21.4) | 66 (27.5) | 60 (24.6) | 0.176 |
| Raucher (ehemals oder aktuell) | 149 (62.1) | 159 (65.4) | 162 (67.5) | 135 (55.3) | 0.030 |
| Akuter Myokardinfarkt mit ST-Hebung | 196 (81.7) | 179 (72.8) | 209 (83.8) | 208 (80.3) | 0.284 |
| Thrombolytika | 143 (59.6) | 126 (51.9) | 143 (59.6) | 119 (48.8) | 0.034 |
| Region des Infarkts | | | | | 0.077 |
| Anterior | 105 (43.8) | 102 (42.0) | 102 (42.5) | 102 (41.8) | |
| Inferior | 103 (42.9) | 89 (36.6) | 98 (40.8) | 83 (34.0) | |
| andere | 32 (13.3) | 52 (21.4) | 40 (16.7) | 58 (23.8) | |
| Killip Klasse bei Aufnahme | | | | | <0.0001 |
| I | 162 | 132 | 114 | 84 (34.4) | |
| | (67.5) | (54.3) | (46.3) | | |
| II | 68 (28.3) | 93 (38.3) | 104 (43.3) | 109 (44.7) | |
| III | 9 (3.8) | 14 (5.8) | 24 (10.0) | 41 (16.8) | |
| IV | 1 (0.4) | 0 (0) | 0 (0) | 9 (3.7) | |
| Peak CK (IU/L) | 838.5± 794.9 | 895.4 ± 1091.8 | 1261.5 ± 1243.1 | 1273.0 ± 1590.7 | <0.0001 |
| eGFR (ml/min/1.73m²) | 76. 9 ±. 16.0 | 74.5 ± 16.1 | 67.7 ± 17.5 | 52.7 ± 18.5 | <0.0001 |
| NTproBNP (pmol/L) | 1031. 6 ± 2267.7 | 1224.9 ± 1783.0 | 2141.5 ± 2400.5 | 4350.6 ± 3547.1 | <0.0001 |

**Tabelle 2: Vergleich von CT-proET-1 und NT-proBNP Konzentrationen in verschiedenen Subgruppen von Patienten mit akutem Myokardinfarkt**

| | Median CT-proET-1 (pmol/L) | p Wert | Median NTproBNP (pmol/L) | p Wert |
|---|---|---|---|---|
| Tod vs. Überleben | 126.0 vs. 74.0 | p<0.0001 | 5929.3 vs.802.4 | p<0.0001 |
| Herzinsuffizienz vs. ohne Ereignis | 112 vs. 74.9 | p<0.0001 | 3932.9 vs. 839.0 | p<0.0001 |
| männlich vs. weiblich | 89.0 vs. 73.0 | p<0.0001 | 788.7 vs. 1632.6 | p<0.0001 |
| früherer AMI vs. kein früherer AMI | 85.0 vs. 75.0 | p<0.0001 | 1.332.3 vs. 844.4 | P<0.001 |
| Hypertension vs. Normotensiv | 84.0 vs. 73.2 | p<0.0001 | 1105.6 vs. 802.4 | p<0.0001 |
| Frühere Herzinsuffizienz vs. keine frühere Herzinsuffizienz | 108.0 vs. 75.9 | P<0.0001 | 4160.1 vs. 856.6 | P<0.0001 |
| STEMI vs. NSTEMI | 78.1 vs. 72.9 | p=NS | 1017.9 vs. 624 . 6 | P<0.002 |
| Killip Klasse über 1 vs. Killip Klasse 1 | 88.9 vs. 70.0 | p<0.0001 | 1595.1 vs. 631.5 | p<0.0001 |

Figur 1: Schema zur diagnostischen Verfügung von CT-proET SEQ ID No. 3 samt dessen Nachweis mittels einer Sonde (Antikörper).
Figur 2: ROC Kurven für die Prognose von Tod/Herzinsuffizienz mittels NT-proBNP oder CT-proET-1 oder der aus dem logistischen Regressionsmodell abgeleiteten Kombination beider Parameter.
Figur 3: Rate der Ereignisse (Tod, Herzinsuffizienz) nach Subgruppierung der Herzinfarktpatienten gemäß der angegebenen Cut-off Konzentrationen für CT-proET-1 und NT-proBNP (beide in pmol/L). Die Cut-off Konzentrationen wurden jeweils als diejenigen Konzentrationen aus der ROC-Analyse ermittelt, bei denen das Produkt aus Sensitivität und Spezifität für die Ereignisprognose maximal war.
Figur 4: Rate der Ereignisse (Tod, Herzinsuffizienz) nach Subgruppierung der Herzinfarktpatienten gemäß Lage der Messwerte für CT-proET-1 und NT-proBNP bezogen auf den jeweiligen Median der Messwerte aller Herzinfarktpatienten. Der Median lag für CT-proET-1 bei 77 pmol/L, für NT-proBNP bei 914 pmol/L.

## Patentansprüche

1. Verfahren zur in-vitro Diagnose und / oder Risikostratifizierung des akuten Koronarsyndroms und /oder post-Myokardinfarkt, **dadurch gekennzeichnet, dass** eine Bestimmung des CT-proET-1 mit den freien Fragmenten SEQ ID No. 2 und / oder SEQ ID No. 3 in Kombination mit NT-proBNP (SEQ ID No. 4) an mindestens einer Patientenprobe eines zu untersuchenden Patienten erfolgt.

2. Verfahren zur Diagnose und / oder Risikostratifizierung des akuten Koronarsyndroms und / oder post-Myokardinfarkt nach Anspruch 1, zur Identifizierung von Patienten mit erhöhtem Risiko oder/und einer ungünstigen Prognose eines akuten Koronarsyndroms, insbesondere Myokardinfarkt, Angina pectoris und / oder post-Myokardinfarkt.

3. Verfahren zur Diagnose und / oder Risikostratifizierung des akuten Koronarsyndroms und / oder post-Myokardinfarkt nach Anspruch 1 oder 2, wobei der Patient ein symptomatischer und / oder asymptomatischer Patient, insbesondere ein Notfallpatient ist.

4. Verfahren zur Diagnose und / oder Risikostratifizierung des akuten Koronarsyndroms und / oder post-Myokardinfarkt nach einem der Ansprüche 1 bis 3
zur Therapiesteuerung des akuten Koronarsyndroms, insbesondere Myokardinfarkt (AMI) und der Angina pectoris (AP) und / oder post-Myokardinfarkt, insbesondere in der Intensivmedizin oder Notfallmedizin.

5. Verfahren zur Diagnose und / oder Risikostratifizierung des akuten Koronarsyndroms und / oder post-Myokardinfarkt nach einem der Ansprüche 1 bis 4 zur Durchführung von klinischen Entscheidungen, insbesondere weiterführende Behandlung und Therapie mittels Arzneimitteln, insbesondere in der Intensivmedizin oder Notfallmedizin, einschließlich der Entscheidung zur Hospitalisierung des Patienten.

6. Verfahren zur Diagnose und / oder Risikostratifizierung des akuten Koronarsyndroms und / oder post-Myokardinfarkt nach einem der Ansprüche 1 bis 4 zur Prognose, zur differentialdiagnostischen Früherkennung und Erkennung, zur Beurteilung des Schweregrades und zur therapiebegleitenden Verlaufsbeurteilung.

7. Verfahren nach einem der vorherigen Ansprüche,
**dadurch gekennzeichnet,**
**dass** die cut-off Werte 80-150 pmol/L, insbesondere 90 - 130 pmol/L insbesondere 109,5 pmol/L für CT-proET-1 und 750 - 1100 pmol/L, insbesondere 800 - 950 pmol/L, insbesondere 827,2 pmol/L für NT-proBNP betragen.

8. Verfahren nach einem der vorherigen Ansprüche,
**dadurch gekennzeichnet,**
**dass** zusätzlich eine Bestimmung mindestens eines weiteren Markers ausgewählt aus der Gruppe inflammatorischer Marker, cardiovaskulärer Marker, neurohormonaler Marker oder ischämischer Marker durchgeführt wird.

9. Verfahren nach einem der vorherigen Ansprüche, **dadurch gekennzeichnet, dass** der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt ist.

10. Verfahren nach einem der vorherigen Ansprüche, **dadurch gekennzeichnet, dass** der cardiovaskuläre Marker aus mindestens einem Marker aus der Gruppe Kreatinkinase, Myoglobin, Myeloperoxidase, natriuretisches Protein, insbesondere ANP (bzw. ANF), proANP, NT-proANP, BNP, proBNP oder jeweils eine Teilsequenz davon, kardiale Troponin, CRP sowie Kreislaufregulierende (Pro)Hormone, wie pro-Gastrin-Releasing Peptid (proGRP), pro-Endothelin-1, pro-Leptin, pro-Neuropeptid-Y, pro-Somatostatin, pro- Neuropeptid-YY, pro-Opiomelanocortin oder pro-Adrenomedullin (proADM) oder jeweils eine Teilsequenz davon ausgewählt ist.

11. Verfahren nach einem der vorherigen Ansprüche, **dadurch gekennzeichnet, dass** der ischämischer Marker aus mindestens einem Marker aus der Gruppe Troponin I und T, CK-MB ausgewählt ist.

12. Verfahren nach einem der vorherigen Ansprüche, **dadurch gekennzeichnet, dass** der neurohormonale Marker mindestens ein natriuretisches Protein ist, insbesondere ANP (bzw. ANF), proANP, NT-proANP, BNP, proBNP oder eine Teilsequenz davon ist.

13. Verfahren nach einem der vorherigen Ansprüche,
**dadurch gekennzeichnet,**
**dass** parallele oder simultane Bestimmungen der Marker durchgeführt werden.

14. Verfahren nach einem der vorherigen Ansprüche,
**dadurch gekennzeichnet,**
**dass** die Bestimmungen an einem Analyseautomaten, insbesondere mittels eines Kryptors, durchgeführt werden.

15. Verfahren nach einem der vorherigen Ansprüche,
**dadurch gekennzeichnet, dass** die Bestimmungen mittels eines Schnelltests, insbesondere in Einzel- oder Multiparameterbestimmungen durchgeführt werden.

16. Verwendung von des CT-proET-1 mit den freien Fragmenten SEQ ID No. 2 und / oder SEQ ID No. 3 in Kombination mit NT-proBNP (SEQ ID No. 4) zur Diagnose und / oder Risikostratifizierung des akuten Koronarsyndroms, Myokardinfarkt, Angina Pectoris und / oder post-Myokardinfarkt und ggfs. weitere Marker nach einem der Ansprüche 8 bis 12.

17. Kit zur Diagnose und / oder Risikostratifizierung des akuten Koronarsyndroms, Myokardinfarkt, Angina Pectoris und / oder post-Myokardinfarkt enthaltend Nachweisreagenzien zur Bestimmung von CT-proET-1 mit den freien Fragmenten SEQ ID No. 2 und / oder SEQ ID No. 3 in Kombination mit NT-proBNP (SEQ ID No. 4) und ggfs. weitere Marker nach einem der Ansprüche 8 bis 12 und Hilfsmittel.

## Claims

1. A method for the in-vitro diagnosis and/or risk classification of acute coronary syndrome, and/or post-myocardial infarction, **characterized in that** a determination of the CT-proET-1 with the free fragments SEQ ID No. 2 and/or SEQ ID No. 3 is carried out in combination with NT-proBNP (SEQ ID No. 4) on at least one patient sample of a patient to be examined.

2. The method for the diagnosis and/or risk classification of acute coronary syndrome, and/or post myocardial infarction according to claim 1, for identifying patients having an increased risk and/or an unfavorable prognosis of acute coronary syndrome, particularly myocardial infarction, Angina pectoris, and/or post-myocardial infarction.

3. The method for the diagnosis and/or risk classification of acute coronary syndrome, and/or post-myocardial infarction according to claims 1 or 2, wherein the patient is a symptomatic and/or asymptomatic patient, particularly an emergency room patient.

4. The method for the diagnosis and/or risk classification of acute coronary syndrome, and/or post-myocardial infarction according to one of the claims 1 to 3 for the therapy control of acute coronary syndrome, particularly myocardial infarction (AMI) and Angina pectoris (AP), and/or post-myocardial infarction, particularly in intensive medicine or emergency medicine.

5. The method for the diagnosis and/or risk classification of acute coronary syndrome, and/or post-myocardial infarction according to one of the claims 1 to 4 for carrying out clinical decisions, particularly the continuative treatment and therapy by means of pharmaceuticals, particularly in intensive medicine or emergency medicine, including the decision for hospitalizing the patient.

6. The method for the diagnosis and/or risk classification of acute coronary syndrome, and/or post-myocardial infarction according to one of the claims 1 to 4 for the prognosis, for the differential diagnostic early detection and recognition, for the assessment of the degree of severity, and for the therapy-accompanying assessment of the course.

7. The method according to one of the previous claims, **characterized in that** the cut-off values are 80-150 pmol/l, particularly 90-130 pmol/l, particularly 109.5 pmol/I for CT-proET-1, and 750-1100 pmol/, particularly 800-950 pmol/l, particularly 827.2 pmol/ for NT-proBNP.

8. The method according to one of the previous claims, **characterized in that** additionally a determination of at least one further marker is carried out, which is selected from the group of inflammatory markers, cardiovascular markers, neurohormonal markers, or ischemic markers.

9. The method according to one of the previous claims, **characterized in that** the inflammatory marker is selected from at least one marker of the group of C-reactive protein (CRP), cytokins, such as TNF-alpha, interleukins, such as IL-6, procalcitonin (1-116, 3-116), and adhesion molecules, such as VCAM or ICAM.

10. The method according to one of the previous claims, **characterized in that** the cardiovascular marker is selected from the group of creatine kinasis, myoglobin, myeloperoxidasis, natriuretic protein, particularly ANP (or ANF), proANP, NT-proANP, BNP, pro-BNP, or a partial sequence thereof, cardial roponin, CRP, and circulation regulating (pro)hormones, such as pro-gastrin-releasing peptide (proGRP), pro-endothelin-1, pro-leptin, pro-neuropeptide-Y, pro-somatostatin, pro-neuropeptide-YY, pro-opiomelanocortin, or pro-adrenomedullin (proADM), or a partial sequence thereof.

11. The method according to one of the previous claims, **characterized in that** the ischemic marker is selected from at least one marker of the group of troponin I and T, CK-MB.

12. The method according to one of the previous claims, **characterized in that** the neurohormonal marker is at least one natriuetic protein, particularly ANP (or ANF), proANP, NT-proANP, BNP, proBNP, or a partial sequence thereof.

13. The method according to one of the previous claims, **characterized in that** the parallel or simultaneous determinations of the marker are to be carried out.

14. The method according to one of the previous claims, **characterized in that** the determinations are carried out on an automatic analysis device, particularly by means of a Kryptor.

15. The method according to one of the previous claims, **characterized in that** the determinations are carried out by means of a quick test, particularly in single or multiple parameter determinations.

16. Use of the CT-proET-1 with the free fragments SEQ ID No. 2 and/or SEQ ID No. 3 in combination with NT-proBNP (SEQ ID No. 4) for the diagnosis and/or risk classification of acute coronary syndrome, myocardial infarction, Angina pectoris, and/or post-myocardial infarction, and optionally further markers according to one of the claims 8 to 12.

17. A kit for the diagnosis and/or risk classification of acute coronary syndrome, myocardial infarction, Angina pectoris, and/or post-myocardial infarction containing detection reagents for determining CT-proET-1 with the free fragments SEQ ID No. 2 and/or SEQ ID No. 3 in combination with NT-proBNP (SEQ ID No. 4) and optionally further markers according to one of the claims 8 to 12, and auxiliary means.

## Revendications

1. Méthode pour le diagnostic in vitro et/ou la classification du risque d'un syndrome coronarien aigu et/ou d'un post-infarctus du myocarde, **caractérisée par le fait qu'**une détermination de la CT-proET-1 avec les fragments libres SEQ ID N° 2 et/ou SEQ ID N° 3 est effectuée en combinaison avec le NT-proBNP (SEQ ID N° 4) sur au moins un échantillon de patient d'un patient à examiner.

2. Méthode pour le diagnostic et/ou la classification du risque d'un syndrome coronarien aigu et/ou d'un post-infarctus du myocarde selon la revendication 1, pour identifier des patients ayant un risque accru et/ou un pronostic non favorable de syndrome coronarien aigu, en particulier un infarctus du myocarde, une angine de poitrine et/ou un post-infarctus du myocarde.

3. Méthode pour le diagnostic et/ou la classification du risque d'un syndrome coronarien aigu et/ou d'un post-infarctus du myocarde selon l'une des revendications 1 ou 2, dans laquelle le patient est un patient symptomatique et/ou asymptomatique, en particulier un patient de salle d'urgence.

4. Méthode pour le diagnostic et/ou la classification du risque d'un syndrome coronarien aigu et/ou d'un post-infarctus du myocarde selon l'une des revendications 1 à 3 pour le contrôle thérapeutique d'un syndrome coronarien aigu, en particulier d'un infarctus du myocarde (IMA) et d'une angine de poitrine (AP), et/ou d'un post-infarctus du myocarde, en particulier en médecine intensive ou en médecine d'urgence.

5. Méthode pour le diagnostic et/ou la classification du risque d'un syndrome coronarien aigu et/ou d'un post-infarctus du myocarde selon l'une des revendications 1 à 4 pour prendre des décisions cliniques, en particulier le traitement complémentaire et la thérapie complémentaire au moyen de produits pharmaceutiques, en particulier en médecine intensive ou en médecine d'urgence, comprenant la décision d'hospitalisation du patient.

6. Méthode pour le diagnostic et/ou la classification du risque d'un syndrome coronarien aigu et/ou d'un post-infarctus du myocarde selon l'une des revendications 1 à 4, pour le pronostic, pour la détection précoce et la reconnaissance précoce par diagnostic différentiel, pour l'évaluation du degré de sévérité et pour l'évaluation d'accompagnement thérapeutique du parcours.

7. Méthode selon l'une des revendications précédentes, **caractérisée par** la fait que les valeurs seuils sont 80-150 pmol/l, en particulier 90-130 pmol/l, en particulier 109,5 pmol/l pour CT-proET-1, et 750-1100 pmol/l, en particulier 800-950 pmol/l, en particulier 827,2 pmol/l pour NT-proBNP.

8. Méthode selon l'une des revendications précédentes, **caractérisée par le fait que**, de plus, une détermination d'au moins un marqueur supplémentaire est effectuée, lequel marqueur est choisi dans le groupe des marqueurs inflammatoires, des marqueurs cardiovasculaires, des marqueurs neurohormonaux ou des marqueurs ischémiques.

9. Méthode selon l'une des revendications précédentes, **caractérisée par le fait que** le marqueur inflammatoire est choisi parmi au moins un marqueur du groupe de la protéine C-réactive (CRP), des cytokines, telles que le TNF-alpha, des interleukines, telles que IL-6, la procalcitonine (1-116, 3-116) et des molécules d'adhésion, telles que VCAM ou ICAM.

10. Méthode selon l'une des revendications précédentes, **caractérisée par le fait que** le marqueur cardiovasculaire est choisi dans le groupe de la créatine kinase, la myoglobine, la myéloperoxidase, la protéine natriurétique, en particulier ANP (ou ANF), proANP, NT-proANP, BNP, pro-BNP ou une séquence partielle de ceux-ci, la roponine cardiaque, CRP et les (pro)hormones de régulation de la circulation, telles qu'un peptide de libération de pro-gastrine (proGRP), la pro-endothéline-1, la proleptine, le pro-neuropeptide-Y, la pro-somatostatine, le pro-neuropeptide-YY, la proopiomélanocortine ou la pro-adrénomédulline (proADM) ou une séquence partielle de ceux-ci.

11. Méthode selon l'une des revendications précédentes, **caractérisée par le fait que** le marqueur ischémique est choisi parmi au moins un marqueur du groupe de la troponine 1 et T, CK-MB.

12. Méthode selon l'une des revendications précédentes, **caractérisée par le fait que** le marqueur neurohormonal est au moins une protéine natriurétique, en particulier ANP (ou ANF), proANP, NT-proANP, BNP, proBNP ou une séquence partielle de ceux-ci.

13. Méthode selon l'une des revendications précédentes, **caractérisée par le fait que** les déterminations parallèles ou simultanées du marqueur doivent être effectuées.

14. Méthode selon l'une des revendications précédentes, **caractérisée par le fait que** les déterminations sont effectuées sur un dispositif d'analyse automatique, en particulier au moyen d'un Kryptor.

15. Méthode selon l'une des revendications précédentes, **caractérisée par le fait que** les déterminations sont effectuées au moyen d'un test rapide, en particulier dans des déterminations de paramètres uniques ou multiples.

16. Utilisation de la CT-proET-1 avec les fragments libres SEQ ID No.2 et/ou SEQ ID No.3 en combinaison au NT-proBNP (SEQ ID No.4) pour le diagnostic et/ou la classification du risque d'un syndrome coronarien aigu, d'un infarctus du myocarde, d'une angine de poitrine et/ou d'un post-infarctus du myocarde et facultativement à d'autres marqueurs selon l'une des revendications 8 à 12.

17. Trousse pour le diagnostic et/ou la classification du risque d'un syndrome coronarien aigu, d'un infarctus du myocarde, d'une angine de poitrine et/ou d'un post-infarctus du myocarde contenant des réactifs de détection pour déterminer CT-proET-1 avec les fragments libres SEQ ID No.2 et/ou SEQ ID No.3 en combinaison avec NT-proBNP (SEQ ID No.4) et facultativement d'autres marqueurs selon l'une des revendications 8 à 12, et des moyens auxiliaires.
